# NEUE EUROPÄISCHE PATENTSCHRIFT

(11) **EP 0 608 285 B2**
(45) Veröffentlichungstag und Bekanntmachung des Hinweises auf die Entscheidung über den Einspruch: **07.04.1999**
(45) Hinweis auf die Patenterteilung: 30.08.1995
(21) Anmeldenummer: 92921085.4
(22) Anmeldetag: 07.10.1992
(51) Int. Cl.: C11D 1/83, C11D 17/00

(54) **VISKOSE WÄSSRIGE TENSIDZUBEREITUNGEN**
VISQUOUS AQUEOUS SURFACE-ACTIVE COMPOSITIONS
PREPARATIONS TENSIOACTIVES VISQUEUSES AQUEUSES

(30) Priorität: 15.10.1991 DE 4134077
(43) Veröffentlichungstag der Anmeldung: 03.08.1994
(73) Patentinhaber: Henkel Kommanditgesellschaft auf Aktien, 40191 Düsseldorf (DE)
(72) Erfinder: NICKEL, Dieter, D-4006 Erkrath 2 (DE); HOFMANN, Rainer, D-4000 Düsseldorf 13 (DE)
(86) Internationale Anmeldenummer: EP9202310
(87) Internationale Veröffentlichungsnummer: WO9308250

(56) Entgegenhaltungen:
- EP-A- 0 092 877
- EP-A- 0 105 556
- EP-A- 0 355 551
- EP-A- 0 370 312
- EP-A- 0 384 983
- EP-A- 0 408 965
- EP-A- 0 486 784
- EP-B- 0 070 076
- WO-A-86/02943
- WO-A-90/02163
- CA-A- 1 180 973
- DE-A- 4 036 663
- US-A- 4 668 422
- D. Balzer, Tenside Bd. 28/6 (1991), s. 419-427

## Beschreibung

Die Erfindung betrifft niedrigkonzentrierte und hochviskose wäßrige Tensidzubereitungen, die Alkylglykoside und bestimmte Aniontenside enthalten, ein Verfahren zu ihrer Herstellung sowie die Verwendung derartiger Zubereitungen als Wasch- oder Reinigungsmittel.

Daß Alkylglykoside mit langkettigen Alkylgruppen zu den nichtionischen Tensiden gehören, ist seit langer Zeit bekannt. Ebenso weiß der Fachmann, wie zum Beispiel in **A.M. Schwartz, J.W. Perry, Surface Active Agents, Vol. I, Interscience Publishers, 1949, Seite 372** beschrieben, daß Tensidmischungen in der Regel synergistische Effekte aufweisen und oft bessere Reinigungseigenschaften besitzen, als sich aus der Summe der Werte der Einzelkomponenten ergeben würde.

Waschmittel, die Alkylglykoside in Kombination mit wenigstens einem üblichen anionischen Tensid im Verhältnis 1 : 10 bis 10 : 1 enthalten, werden in der europäischen Patentanmeldung **EP 070 074** beschrieben. Waschmittel, die Alkylglykoside und Aniontenside enthalten, sind auch aus der europäischen Patentanmeldung **EP 092 877** bekannt. Des weiteren sind aus der europäischen Patentanmeldung **EP 105 556** flüssige Waschmittel bekannt, die Alkylglykoside, bestimmte andere nichtionische Tenside und anionische Tenside enthalten. Aus der internationalen Patentanmeldung **WO 86/02943** sind alkylglykosidhaltige Flüssigiwaschmittel bekannt, die übliche Aniontenside enthalten. Aus der europäischen Patentanmeldung **EP 132 043** ist ein Verfahren zur Herstellung von Alkylglykosiden unter Einsatz katalytischer Mengen eines Aniontensids in dessen Säureform bekannt. In der europäischen Patentanmeldung **EP 132 046** wird vorgeschlagen, ein derartiges Herstellungsverfahren durch Zugabe bestimmter Basen nach der eigentlichen Reaktion zur Neutralisation des Katalysators zu modifizieren. Die in diesen Dokumenten erwähnten Wasch- beziehungsweise Reinigungsmittelvorprodukte auf Alkylglykosid-Basis sind relativ hochkonzentrierte wäßrige Lösungen beziehungsweise Pasten, da die für die Mischung zu fertigen Mitteln vorgesehenen Komponenten einen möglichst hohen Aktivsubstanzgehalt aufweisen sollen. Gleichzeitig müssen sie leicht handhabbar sein, das heißt, sie sollten eine möglichst niedrige Viskosität aufweisen, fließfähig und leicht pumpbar sein. Demgegenüber wird an vom Verbraucher angewendete wäßrige Flüssigprodukte, zu denen insbesondere Flüssigwaschmittel, Geschirrspülmittel und Universalreiniger, aber auch kosmetische Produkte, beispielsweise Haarshampoos oder Körperreinigungslotionen gehören, die Forderung an eine gewisse Mindestviskosität gestellt, obwohl der Aktivsubstanzgehalt derartiger Produkte in der Regel relativ niedrig ist. Derartige Produkte enthalten daher normalerweise Verdickungsmittel, die in der Regel den Nachteil ausweisen, selbst nichts zur Reinigungsleistung der in den Mitteln enthaltenen Tensidkomponente beizutragen. Übliche Verdickungsmittel sind anorganische wasserlösliche Salze, insbesondere Natriumchlorid, und Salze nicht oberflächenaktiver aromatischer Sulfonsäuren, beispielsweise Natrium-Cumolsulfonat.

Organische Verdickungsmittel sind üblicherweise Fettsäurealkanolamide, wie Kokosfettsäuremonoethanolamid, Laurinsäuremonoethanolamid, Ölsäurediethanolamid und Kokosfettsäurediethanolamid, Polyethylenglykoldifettsäureester sowie eine Reihe wasserlöslicher Polymere.

In den meisten Fällen ist es nicht oder nur unter Einsatz großer Konzentration möglich, allein durch Verwendung anorganischer Salze die gewünschte Viskosität in der Tensidlösung aufzubauen. Man ist daher in der Regel gewungen, zusätzlich zu den anorganischen Salzen organische Verdickungsmittel einzusetzen, die mit einer Reihe von Nachteilen behaftet sind. So weisen mit Polyethylenglykolfettsäurediestern verdickte Lösung oft eine unzureichende Viskositätsstabilität bei Lagerung auf, während wasserlösliche Polymere sich oft nur schwer auflösen und ein unerwünschtes Fließverhalten bewirken.

In der europäischen Patentanmeldung **EP 306 843** wird vorgeschlagen, zur Verdickung von Tensidlösungen Anlagerungsprodukte von Ethylenoxid und/oder Propylenoxid an ungesättigte Fettalkohole zu verwenden. Diese Produkte weisen die oben genannten Nachteile nicht mehr auf; es besteht jedoch nach wie vor das Bedürfnis nach Verdickungsmitteln mit vergleichbaren Eigenschaften bei erhöhter verdickender Wirkung, Dies würde bei gegebenem Gehalt an Verdickungsmitteln die Herstellung von Systemen mit höherer Viskosität ebenso ermöglichen wie die Verringerung des Gehaltes an Verdikkungsmitteln bei vorgegebener Viskosität.

Die europäische Patentanmeldung **EP-A-0 355 551** offenbart ein pastenförmiges Wasch- und Reinigungsmittel enthaltend eine Tensidkombination aus einem Alkylglykosid als nichtionisches Tensid und einem α-Sulfofettsäuresalz als anionisches Tensid, im Mengenverhältnis 10:1 bis 1:3.

Der vorliegenden Erfindung lag die Aufgabe zugrunde, eine lagerstabile, flüssige, fließfähige Alkylglykosid-Zubereitung auf wäßriger Basis zu entwickeln, die durch ihre relativ hohe Viskosität eine leichte Handhabbarkeit unter Anwendungsbedingungen aufweist und möglichst geringe Mengen an nicht-alkylglykosidischer Aktivsubstanz enthält.

Diese Aufgabe wird durch eine wäßrige Mischung aus bestimmten Mengen an Alkylglykosid und bestimmtem Aniontensid gelöst.

Die erfindungsgemäßen Tensidzubereitungen sind gelförmige Tensidzubereitungen, bestehend aus
(a) 2 bis 12 Gew.-% Alkylglykosiden der Formel (I),

   R¹O-[G]ₙ (I)

   in der R¹ für einen Alkylrest mit 8 bis 22 Kohlenstoffatomen, G für eine Glykose-Einheit und n für Zahlen von 1 bis 10 steht,
(b) 0.05 bis 2 Gew.-% Aniontensiden, ausgewählt aus der Gruppe, die gebildet wird von Alkylsulfaten, Alkansulfonaten, Estern von α-Sulfofettsäuren mit Alkoholen mit 1 bis 4 Kohlenstoffatomen, Sulfonierungsprodukten von Alkenen oder ungesättigten Fettsäuren sowie deren Gemischen, und
(c) ad 100 Gew.-% Wasser,
mit der Maßgabe, daß das Gewichtsverhältnis der Komponenten (a) und (b) im Bereich 40 :1 bis 5:1 liegt.

Unter gelförmigen Zubereitungen werden im Rahmen der vorliegenden Erfindung strukturierte, isotrope, viskoelastische Lösungen mit einer aus einer aus Stäbchenmicellen aufgebauten viskoelastischen Netzwerkstruktur verstanden. Sie weisen vorzugsweise eine Viskosität, gemessen bei Temperaturen von 20 °C bis 25 °C bei einer Scherrate von 10 s⁻¹, von 250 mPa·s bis 10 0000 mPa·s, insbesondere von 600 mPa·s bis 7 000 mPa·s, auf.

Vorzugsweise enthalten die erfindungsgemäßen Alkylglykosid-Zubereitungen 3 Gew.-% bis 12 Gew.-%, insbesondere 4 Gew.-% bis 10 Gew.-% Alkylglykosid gemäß Formel I, 0,075 Gew.-% bis 2 Gew.-%, insbesondere 0,1 Gew.-% bis 2 Gew.-% Aniontensid und auf 100 Gew.-% Wasser. Ein Teil des Wassers, vorzugsweise nicht mehr als 2 Gew.-%, bezogen auf erfindungsgemäße Zubereitung, kann durch ein wassermischbares organisches Lösungsmittel, vorzugsweise aus der Gruppe umfassend Alkohole mit 1 bis 4 C-Atomen, Glykole mit 2 bis 4 C-Atomen und die aus diesen ableitbaren Di- und Triglykole sowie die entsprechenden Glykolether und Gemische der genannten Substanzen, ersetzt werden.

Ein weiterer Gegenstand der Erfindung ist ein Verfahren zur Herstellung lagerstabiler Tensidzubereitungen auf wäßriger Basis, die bei Temperaturen von 20 °C bis 25 °C und einer Scherrate von 10 s⁻¹ eine Viskosität im Bereich von 250 mPa.s bis 10 000 mPa.s aufweisen, durch Mischen einer wäßrigen Lösung, die ein Alkylglykosid der Formel I,

R¹-O(G)ₙ (I)

in der R¹ einen Alkylrest mit 8 bis 22 C-Atomen, G eine Glykose-Einheit und n eine Zahl zwischen 1 und 10 bedeuten, mit einem Aniontensid ausgewählt aus der Gruppe, die gebildet wird von Alkylsulfaten, Alkansulfonaten, Estern von α-Sulfofettsäuren mit Alkoholen mit 1 bis 4 Kohlenstoffatomen, Sulfonierungsprodukten von Alkenen oder ungesättigten Fettsäuren sowie deren Gemischen, im Gewichtsverhältnis von Alkylglykosid gemäß Formel I zu Aniontensid von 40:1 bis 5:1, und mit so viel Wasser mischt, daß der Gesamttensidgehalt in der Zubereitung 2,5 bis 10 Gew.-% beträgt.

Dabei geht man vorzugsweise derart vor, daß man eine wäßrige Paste, welche Alkylglykosid gemäß Formel I in einer Konzentration von 30 Gew.-% bis 60 Gew.-% enthält, in einer solchen Menge mit einer wäßrigen Lösung mischt, die mindestens ein genanntes Aniontensid enthält, daß die Summe der Tenside in der resultierenden wäßrigen Zubereitung 2,5 Gew.-% bis 10 Gew.-% beträgt und das resultierende Gel 90 Gew.-% bis 97,5 Gew.-% Wasser enthält. Das Wasser kann im Prinzip an jeder Stelle der Herstellung der erfindungsgemäßen Mittel zugemischt werden, falls die eingesetzten Ausgangsmaterialien nicht schon genügend derartiges Lösungsmittel enthalten. Vorzugsweise werden die Komponenten auf Temperaturen von 60 °C bis 80 °C erwärmt und mit üblichen Mischern, die vorzugsweise beheizbar sind und während des gesamten Mischvorgangs insbesondere bei Temperaturen im genannten Bereich gehalten werden, vermischt.

Die für die erfindungsgemäßen Tensidzubereitungen geeigneten Alkylglykoside und ihre Herstellung werden zum Beispiel in den europäischen Patentanmeldungen **EP 92 355, EP 301 298, EP 357 969** und **EP 362 671** oder der US-amerikanischen Patentschrift **US 3 547 828** beschrieben. Bei den Glykosidkomponenten ((G)ₙ in Formel I) derartiger Alkylglykoside handelt es sich um Oligo- oder Polymere aus natürlich vorkommenden Aldose- oder Ketose-Monomeren, zu denen insbesondere Glucose, Mannose Fruktose, Galaktose, Talose, Gulose, Altrose, Allose, Idose, Ribose, Arabinose, Xylose und Lyxose gehören. Die aus derartigen glykosidisch verknüpften Monomeren bestehenden Oligomere werden außer durch die Art der in ihnen enthaltenen Zucker durch deren Anzahl, den sogenannten Oligomerisierungsgrad, charakterisiert. Der Oligomerisierungsgrad (n in Formel I) kann als analgetisch zu ermittelnde Größe auch gebrochene Zahlenwerte annehmen; er liegt in der Regel bei Werten zwischen 1 und 10, bei den vorzugsweise eingesetzten Alkylglykosiden unter einem Wert von 1,5, insbesondere zwischen 1,2 und 1,4. Bevorzugter Monomer-Baustein ist wegen der guten Verfügbarkeit Glucose.

Der Alkylteil (R¹ in Formel I) der in den erfindungsgemäßen Tensidzubereitungen enthaltenen Alkylglykoside stammt bevorzugt ebenfalls aus leicht zugänglichen Derivaten nachwachsender Rohstoffe, insbesondere aus Fettalkoholen, obwohl auch deren verzweigtkettige Isomere, insbesondere sogenannte Oxoalkohole, zur Herstellung verwendbarer Alkylglykoside eingesetzt werden können. Brauchbar sind demgemäß insbesondere die primären Alkohole mit linearen Octyl-, Decyl-, Dodecyl-, Tetradecyl-, Hexadecyl- oder Octadecylresten sowie deren Gemische. Besonders geeignete Alkylglykoside enthalten einen Kokosfettalkylrest, das heißt Mischungen mit im wesentlichen R¹=Dodecyl und R¹=Tetradecyl.

Die Alkylglykoside können herstellungsbedingt geringe Mengen, beispielsweise 1 bis 2 %, an nicht umgesetztem freiem Fettalkohol enthalten, was sich in der Regel nicht nachteilig auf die Eigenschaften der damit hergestellten Zubereitungen auswirkt.

Die im Rahmen der Erfindung einsetzbaren Alkylsulfate sind bekannte anionische Tenside, die in der Regel durch Umsetzung von aliphatischen primären Alkoholen mit einem Sulfatierungsreagenz, beispielsweise Schwefeltrioxid oder Chlorsulfonsäure sowie nachtfolgender Neutralisation und Hydrolyse der entstandenen Reaktionsprodukte, vorzugsweise mit Alkali-, Ammonium- oder Alkyl- beziehungsweise Hydroxyalkyl-substituiuerten Ammoniumbasen, hergestellt werden, Alkylsulfate, auf die sich das erfindungsgemäße Verfahren erstreckt, leiten sich vorzugsweise von Fettalkoholen mit 12 bis 22 Kohlenstoffatomen, insbesondere 12 bis 18 Kohlenstoffatomen, ab. Typische Beispiele hierfür sind Laurylalkohol, Myristylalkohol, Cetylalkohol, Stearylalkohol oder Behenylalkohol. Die Alkylsulfate können sich auch von technischen Alkoholgemischen ableiten, wie sie zum Beispiel bei der Hydrierung von technischen Fettsäureestergemischen natürlicher Herkunft oder von Aldehyden aus der Roelen'schen Oxosynthese anfallen. Bevorzugt sind hierbei Alkylsulfate auf der Basis von technischen Kokos- oder Talgalkoholschnitten.

Derartige Alkylsulfate können auch, bedingt durch den Anteil ungesättigter Alkohole im Einsatzmaterial für die Sulfatierung, mehr oder weniger große Mengen an Sulfierungsprodukten von ein-, zwei- oder dreifach ungesättigten Alkoholen der genannten Kettenlängen enthalten. Dabei handelt es sich, da bei Einsatz ungesättigter Alkohole bei der Sulfatierung auch eine Anlagerung des Sulfatierungsmittels an die Doppelbindung stattfinden kann, in der Regel um Mischungen aus Alkenylsulfaten mit Stoffen, die eine innenständige Sulfonatgruppe beziehungsweise eine Sulfonat- und eine Sulfatgruppe enthalten. Der Begriff "Alkylsulfat" umfaßt daher im Rahmen der vorliegenden Erfindung auch derartige Gemische. Vorzugsweise sind aus ungesättigten Alkoholen hervorgegangene Sulfierungsprodukte, wenn im Alkylsulfat vorhanden, in Mengen nicht über 80 Gew.-%, insbesondere von 30 Gew.-% bis 70 Gew.-%, im eingesetzten Alkylsulfat, bezogen auf gesamte in diesem enthaltene aniontensidische Aktivsubstanz, enthalten.

Die für die Einarbeitung in die erfindungsgemäßen Zubereitungen geeigneten Sulfofettsäuresalze sind neutralisierte Derivate der mindestens eine Doppelbindung enthaltenden Fettsäuren mit 8 bis 22 C-Atomen. Zu diesen gehören insbesondere die Sulfonierungsprodukte von Lauroleinsäure, Myristoleinsäure, Palmitoleinsäure, Ölsäure, Gadoleinsäure und Erucasäure. Derartige Sulfofettsäuresalze werden durch Umsetzung der ungesättigten Fettsäuren mit einem Sulfonierungsmittel und anschließende Neutralisation und Hydrolyse mit den genannten wäßrigen Basen nach bekannten Verfahren, wie sie zum Beispiel in der britischen Patentschrift **GB 1 278 421** oder der internationalen Patentanmeldung **WO 90/06300** beschrieben sind, hergestellt. Dabei entstehen Gemische der eine Sulfo- und eine Hydroxygruppe tragenden gesättigten Fettsäuren mit durch formale Eliminierung eines Molequivalents Wasser aus diesen entstehenden ungesättigten, eine Sulfogruppe tragenden Fettsäuren. Durch analoge Reaktion können aus Alkenen mit vorzugsweise 12 bis 22 C-Atomen oberflächenaktive Gemische aus Hydroxyalkylsulfonaten mit Alkensulfonaten erzeugt werden.

Die erfindungsgemäß brauchbaren α-Sulfofettsäureester können durch Umsetzung der Ester von Fettsäuren, die 6 bis 22, vorzugsweise 16 bis 18 Kohlenstoffatome enthalten und vorzugsweise eine lodzahl kleiner 20 aufweisen, mit C₁ bis C₄-Alkoholen, insbesondere Fettsäuremethylester, mit einem sulfiermittel und nachfolgende Neutralisation mit wäßrigen Basen hergestellt werden. Als Sulfiermittel kommen in diesem Fall insbesondere Schwefelsäure, Oleum, Chlorsulfonsäure oder gasförmiges Schwefeltrioxid im Gemisch mit einem Inertgas in Betracht. Als Neutralisationsbasen eignen sich vor allem wäßrige Lösungen ion Alkali- und Erdalkalihydroxiden oder Ammoniak Typische Beispiele für Fettsäuren der genannten Art sind Capronsäure, Caprylsäure, Caprinsäure, Laurinsäure, Myristinsäure oder Behensäure. Substanzen mit besonders günstigen Detergenseigenschatlen werden auf Basis von Palmitinund Stearinsäure erhalten, die aus diesen Gründen bevorzugt sind. Diese Fettsäuren können auch in Form technischer Gemische vorliegen, wie sie üblicherweise bei der Spaltung pflanzlicher oder tierischer Fettsäureglycerinester anfallen. Weisen derartige Gemische hohe Anteile ungesättigter Fettsäuren auf, können sie in in sich bekannter Weise durch Hydrierung z. B. in Gegenwart von Nickelkatalysatoren in Gemische weitgehend gesättigter Fettsäuren überführt werden, deren Iodzahl kleiner 20 ist.

Bei den erfindungsgemäß einsetzbaren Alkansulfonaten handelt es sich um Substanzen, die durch Sulfoxidation von Kohlenwasserstoffen, welche vorzugsweise 10 bis 20 C-Atome enthalten, gewonnen werden. Dabei entstehen in der Regel Produkte mit statistischer Verteilung der Sulfonsäure-Substituenten, die gewünschtenfalls in bekannter Weise getrennt werden können. Für die erfindungsgemäßen Mischungen sind sekundäre Alkansulfonate mit 12 bis 17 C-Atomen besonders geeignet. Als Kationen kommen auch in diesem Fall insbesondere solche aus der Gruppe der Alkaliionen, Ammonium- oder Alkyl- beziehungsweise Hydroxyalkyl-substituierten Ammoniumionen in Betracht.

Die genannten Aniontenside sind seit langer Zeit als gut wirksam bekannt. Überraschend war allerdings, daß durch den Zusatz sehr geringer Mengen derartiger Aniontenside die Viskosität verdünnter wäßriger Alkylglykosid-Lösungen deutlich erhöht werden kann. Im Gegensatz dazu nimmt die Viskosität verdünnter wäßriger Alkylglykosid-Lösungen durch den Zusatz anderer Aniontenside, beispielsweise Alkylbenzolsulfonat, ab. Im Rahmen der vorliegenden Erfindung sind die Alkylbenzolsulfonate, obwohl bekanntermaßen sehr wirksame Aniontenside, daher nicht brauchbar.

Die erfindungsgemäßen Tensidzubereitungen weisen ausgezeichnete Reinigungseigenschaften und eine hohe Kaltwasserlöslichkeit auf. Sie werden daher vorzugsweise als Wasch-, Spül- oder Reinigungsmittel sowie in der Haar- und Körperpflege verwendet.

### Beispiele

### Beispiel 1

Wäßrige Mischungen aus den in den Tabellen 1 (Summe der Tensidkomponenten 5 Gew.-%) und 2 (Summe der Tensidkomponenten 10 Gew.-%) angegebenen Aniontensiden mit C_{12/14}-Alkylglucosid (Oligomerisierungsgrad 1,4) in den angegebenen Mischungsverhältnissen (Alkylglykosid zu Aniontensid) wiesen folgende Viskositäten (25 °C, gemessen mit einem schubspannungskontrollierten Rotationsrheometer Carri-Med^{(R)} CS 100 bei einer Scherrate von 10 s⁻¹) auf:

**Tabelle 1:**

| Viskosität 5-gewichtsprozentiger Zubereitungen (25 °C) | | |
|---|---|---|
| Aniontensid | Mischungsverhältnis [Gew.-%] | Viskosität [mPa.s] |
| - | 5:0 | 320 |
| Sulfopon T^{a)} | 4,9:0,1 | 1090 |
| | 4,75:0,25 | 1560 |
| | 4,5:0,5 | 1560 |
| Sulfopon K^{b)} | 4,9:0,1 | 770 |
| | 4,5:0,5 | 1000 |
| Sulfoester^{c)} | 4,9:0,1 | 620 |
| | 4,75:0,25 | 1780 |
| | 4,5:0,5 | 1200 |
| Na-C₁₆-Alkysulfat | 4,9:0,1 | 930 |
| | 4,75:0,25 | 1940 |
| | 4,5:0,5 | 4530 |

| | | |
|---|---|---|
| a) : Na-C_{16/18}-Alkylsulfat, Hersteller Henkel | | |
| b) : Na-Laurylsulfat, Hersteller Henkel | | |
| c) : Na-Salz eines α-sulfonierten C_{16/18}-Fettsäuremethylestergemischs, Hersteller Henkel | | |

**Tabelle 2:**

| Viskosität 10-gewichtsprozentiger Zubereitungen (25 °C) | | |
|---|---|---|
| Aniontensid | Mischungsverhältnis [Gew.-%] | Viskosität [mPa.s] |
| - | 10:0 | 720 |
| Sulfopon T^{a)} | 9,9:0,1 | 1010 |
| | 9,5:0,5 | 2450 |
| | 9:1 | 4380 |
| Sulfopon K^{b)} | 9,9:0,1 | 830 |
| | 9,5:0,5 | 1730 |
| | 9:1 | 4980 |
| Sulfoester^{c)} | 9,9:0,1 | 830 |
| | 9,5:0,5 | 1890 |
| | 9:1 | 6950 |
| Na-C₁₆-Alkylsulfat | 9,9:0,1 | 1340 |
| | 9,75:0,25 | 1510 |
| | 9,5:0,5 | 1780 |
| | 9:1 | 4300 |

| | | |
|---|---|---|
| a) : Na-C_{16/18}-Alkylsulfat, Hersteller Henkel | | |
| b) : Na-Laurylsulfat, Hersteller Henkel | | |
| c) : Na-Salz eines α-sulfonierten C_{16/18}-Fettsäuremethylestergemischs, Hersteller Henkel | | |

Zum Vergleich wurden nicht erfindungsgemäße Zubereitungen hergestellt und getestet, die C_{12/14}-Alkylglucosid und Na-C_{11/13}-Alkylbenzolsulfonat (Maranil^{(R)} Hersteller Hüls) enthielten. Diese wiesen in 5-gewichtsprozentiger Lösung (Gewichtsverhältnis 4,9:0,1) eine Viskosität von 270 mPa·s und in 10-gewichtsprozentiger Lösung (Gewichtsverhältnis 9:1) eine Viskosität von 360 mPa·s auf.

## Patentansprüche

1. Gelförmige Tensidzubereitungen, bestehend aus
(a) 2 bis 12 Gew.-% Alkylglykosiden der Formel (I),
R¹O-[G]ₙ (I)
in der R¹ für einen Alkylrest mit 8 bis 22 Kohlenstoffatomen, G für eine Glykose-Einheit und n für Zahlen von 1 bis 10 steht,
(b) 0,05 bis 2 Gew.-% Aniontensiden, ausgewählt aus der Gruppe, die gebildet wird von Alkylsulfaten, Alkansulfonaten, Estern von α-Sulfofettsäuren mit Alkoholen mit 1 bis 4 Kohlenstoffatomen, Sulfonierungsprodukten von Alkenen oder ungesättigten Fettsäuren sowie deren Gemischen, und
(c) ad 100 Gew.-% Wasser,
mit der Maßgabe, daß das Gewichtsverhältnis der Komponenten (a) und (b) im Bereich 40 :1 bis 5:1 liegt.

2. Tensidzubereitung nach Anspruch 1, **dadurch gekennzeichnet,** daß sie eine Viskosität, gemessen bei Temperaturen von 20 °C bis 25 °C bei einer Scherrate von 10 s⁻¹, von 250 mPas bis 10 000 mPas, insbesondere von 600 mPas bis 7 000 mPas aufweist.

3. Tensidzubereitung nach Anspruch 1 oder 2, **dadurch gekennzeichnet,** daß das Aniontensid ein Alkylsulfat mit einen linearen Alkylrest mit 12 bis 18 C-Atomen enthält.

4. Tensidzubereitung nach Anspruch 3, **dadurch gekennzeichnet,** daß das Alkylsulfat, bezogen auf gesamte in diesem enthaltene aniontensidische Aktivsubstanz, nicht mehr als 80 Gew.-%, insbesondere 30 Gew.-% bis 70 Gew.-%, Sulfierungsprodukte ungesättigter Alkohole enthält.

5. Tensidzubereitung nach Anspruch 1 oder 2, **dadurch gekennzeichnet,** daß das Aniontensid ein Alkansulfonat mit 10 bis 20 C-Atomen, insbesondere ein sekundäres Alkansulfonat mit 12 bis 17 C-Atomen, enthält.

6. Tensidzubereitung nach Anspruch 1 oder 2, **dadurch gekennzeichnet,** daß das Aniontensid ein neutralisiertes Sulfonierungsprodukt einer mindestens eine Doppelbindung enthaltenden Fettsäure mit 8 bis 22 C-Atomen enthält.

7. Tensidzubereitung nach Anspruch 1 oder 2, **dadurch gekennzeichnet,** daß das Aniontensid einen α-Sulfofettsäureester mit 6 bis 22, insbesondere 16 bis 18 C-Atomen enthält.

8. Tensidzubereitung nach Anspruch 1 oder 2, **dadurch gekennzeichnet,** daß das Aniontensid ein neutralisiertes Sulfonierungsprodukt eines Alkens mit 12 bis 22 C-Atomen enthält.

9. Verfahren zur Herstellung lagerstabiler gelförmiger Tensidzubereitungen auf wäßriger Basis, die bei Temperaturen von 20 °C bis 25 °C und einer Scherrate von 10 s⁻¹ eine Viskosität im Bereich von 250 mPa.s bis 10 000 mPa.s aufweisen, durch Mischen einer wäßrigen Lösung, die ein Alkylglykosid der Formel I,
R¹-O(G)ₙ (I)
in der R¹ einen Alkylrest mit 8 bis 22 C-Atomen, G eine Glykose-Einheit und n eine Zahl zwischen 1 und 10 bedeuten, mit einem Aniontensid ausgewählt aus der Gruppe, die gebildet wird von Alkylsulfaten, Alkansulfonaten, Estern von α-Sulfofettsäuren mit Alkoholen mit 1 bis 4 Kohlenstoffatomen, Sulfonierungsprodukten von Alkenen oder ungesättigten Fettsäuren sowie deren Gemischen, im Gewichtsverhältnis von Alkylglykosid gemäß Formel I zu Aniontensid von 40:1 bis 5:1, und mit so viel Wasser mischt, daß der Gesamttensidgehalt in der Zubereitung 2,5 bis 10 Gew.-% beträgt.

10. Verwendung einer Tensid-Zubereitung gemäß einem der Ansprüche 1 bis 8 als Wasch,-Spül-, Reinigungs-, Haar- oder Körperpflegemittel.

## Claims

1. Gel-like surfactant preparation consisting of: 2% by weight to 12% by weight of an alkyl glycoside corresponding to formula I
R¹-O(G)ₙ (I)
in which R¹ is an alkyl radical containing 8 to 22 carbon atoms, G is a glycose unit and n is a number of 1 to 10, 0.05% by weight to 2% by weight of an anionic surfactant of the sulfate or sulfonate type selected from the group consisting of alkyl sulfates, alkane sulfonates, esters of α-sulfofatty acids with C₁₋₄ alcohols, sulfonation products of alkenes or unsaturated fatty acids and mixtures thereof, the ration by weight of alkyl glycoside corresponding to formula I to anionic surfactant being 40:1 to 5:1, and ad 100% by weight water.

2. A surfactant preparation as claimed in claim 1, characterized in that it has a viscosity, as measured at temperatures of 20°C to 25°C at a shear rate of 10 s⁻¹, in the range from 250 mPas to 10,000 mPas and, more particularly, in the range from 600 mPas to 7,000 mPas.

3. A surfactant preparation as claimed in claim 1 or 2, characterized in that the anionic surfactant contains an alkyl sulfate with a linear alkyl component containing 12 to 18 carbon atoms.

4. A surfactant preparation as claimed in claim 3, characterized in that the alkyl sulfate contains no more than 80% by weight and, more particularly, from 30% by weight to 70% by weight sulfonation products of unsaturated alcohols, based on the total anionic surfactant active substance present therein.

5. A surfactant preparation as claimed in claim 1 or 2, characterized in that the anionic surfactant contains an alkane sulfonate containing 10 to 20 carbon atoms and, more particularly, a secondary alkane sulfonate containing 12 to 17 carbon atoms.

6. A surfactant preparation as claimed in claim 1 or 2, characterized in that the anionic surfactant contains a neutralized sulfonation product of a C₈₋₂₂ fatty acid containing at least one double bond.

7. A surfactant preparation as claimed in claim 1 or 2, characterized in that the anionic surfactant contains an α-sulfofatty acid ester containing 6 to 22 carbon atoms and, more particularly, 16 to 18 carbon atoms.

8. A surfactant preparation as claimed in claim 1 or 2, characterized in that the anionic surfactant contains a neutralized sulfonation product of an alkene containing 12 to 22 carbon atoms.

9. A process for the production of storable, water-based gel-form surfactant preparations, which have a viscosity of 250 mPa.s to 10,000 mPa.s at temperatures of 20 to 25°C by mixing an aqueous solution containing an alkyl glycoside corresponding to formula I
R¹-O(G)ₙ (I)
in which R¹ is an alkyl radical containing 8 to 22 carbon atoms, G is a glycose unit and n is a number of 1 to 10, with an anionic surfactant of the sulfate or sulfonate type selected from the group consisting of alkyl sulfates, alkane sulfonates, esters of α-sulfofatty acids with C₁₋₄ alcohols, sulfonation products of alkenes or unsaturated fatty acids and mixtures thereof in a ratio by weight of alkyl glycoside corresponding to formula I to sulfate and/or sulfonate of 40:1 to 5:1 and with water in such a quantity that the total surfactant content in the preparation is 2.2% by weight to 15% by weight.

10. The use of the surfactant preparation claimed in any of claims 1 to 8 as a laundry detergent, dishwashing detergent, cleaning preparation, hair-care or body-care preparation.

## Revendications

1. Gélifié préparation de tensioactifs constitué de :
- 2 % en poids à 12 % en poids d'un alkylglycoside de formule I :
R¹-O(G)ₙ (I)
dans laquelle R¹ est un radical alkyle de 8 à 22 atomes de C, G est une unité glucose et n un nombre de 1 à 10,
- 0,05 % en poids à 2 % en poids d'un tensioactif anionique du type sulfate ou sulfonate, choisi dans le groupe comprenant des alkylsulfates, des alcanesulfonates, des esters des α-sulfoacides gras avec des alcools C₁-C₄, des produits de sulfonation d'alcènes ou d'acides gras insaturés ainsi que leurs mélanges, la proportion pondérale de l'alkylglycoside de formule I au tensioactif anionique représente 40:1 à 5:1, et
- ad 100 % en poids d'eau.

2. Préparation de tensioactifs selon la revendication 1, caractérisée en ce qu'elle présente une viscosité mesurée à des températures de 20°C à 25°C par un gradient de cisaillement de 10 s⁻¹, de 250 mPa.s à 7.000 mPa.s.

3. Préparation de tensioactifs selon la revendication 1 ou 2, caractérisée en ce que le tensioactif anionique contient un alkylsulfate avec un radical alkyle linéaire de 12 à 18 atomes de C.

4. Préparation de tensioactifs selon la revendication 3, caractérisée en ce que l'alkylsulfate, rapporté à l'ensemble de la substance active tensioactive anionique qui le renferme, ne contient pas plus de 80 % en poids, notamment de 30 % en poids à 70 % en poids de produits de sulfatation d'alcools insaturés.

5. Préparation de tensioactifs selon la revendication 1 ou 2, caractérisée en ce que le tensioactif anionique contient un alcanesulfonate de 10 à 20 atomes de C, notamment un alcanesulfonate secondaire de 12 à 17 atomes de C.

6. Préparation de tensioactifs selon la revendication 1 ou 2, caractérisée en ce que le tensioactif anionique contient un produit de sulfonation neutralisé d'un acide gras contenant au moins une double liaison avec 8 à 22 atomes de C.

7. Préparation de tensioactifs selon la revendication 1 ou 2, caractérisée en ce que le tensioactif anionique contient un ester de α-sulfoacide gras ayant chacun de 6 à 22, notament 16 à 18 atomes de C.

8. Préparation de tensioactifs selon la revendication 1 ou 2, caractérisée en ce que le tensioactif anionique contient un produit de sulfonation neutralisé d'un alcène de 12 à 22 atomes de C.

9. Procédé de production de préparations de tensioactifs gélifiées, stables au stockage, qui présentent à des températures de 20 à 25°C et avec un gradient de cisaillement de 10ₛ⁻¹ une viscosité comprise entre 250 mPa.s et 10.000 mPa.s, par mélange d'une solution aqueuse qui contient un alkylglycoside de formule I :
R¹ -O(G)ₙ (I)
dans laquelle R¹ est un radical alkyle de 8 à 22 atomes de C, G est une unité glycose et n un nombre de 1 à 10,
- 0,05 % en poids à 2 % en poids d'un tensioactif anionique du type sulfate ou sulfonate, choisi dans le groupe comprenant des alkylsulfates, des alcanesulfonates, des esters des α-sulfoacides gras avec des alcohols C₁-C₄, des produits de sulfonation d'alcènes ou d'acides gras insaturés ainsi que leurs mélanges, la proportion pondérale de l'alkylglycoside de formule I au sulfate et/ou sulfonate représente 40:1 à 5:1, et on mélange avec suffisamment d'eau, pour que la teneur totale en tensioactif dans la préparation représente 2,5 % en poids à 10 % en poids.

10. Utilisation d'une préparation de tensioactifs selon l'une des revendications 1 à 8, comme produit pour le lavage, le rinçage, le nettoyage, les soins du corps et des cheveux.
